# EUROPEAN PATENT APPLICATION

(11) **EP 2 949 744 A1**
(43) Date of publication of application: **02.12.2015**
(21) Application number: 15169363.7
(22) Date of filing: 27.05.2015
(51) Int. Cl.: C12M 1/00, C12M 1/36

(54) **MEDIUM EXCHANGE SYSTEM**

(30) Priority: 30.05.2014 JP 2014111856
(71) Applicant: OLYMPUS CORPORATION, Shibuya-ku Tokyo 151-0072 (JP)
(72) Inventor: Kimura, Hiroyuki, Tokyo, Tokyo 151-0072 (JP); Minami, Tatsuya, Tokyo, Tokyo 151-0072 (JP); Makara, Yasunori, Tokyo, Tokyo 151-0072 (JP)
(74) Representative: Schicker, Silvia

(57) **Abstract**

Supply and discharge of a solution, such as a medium, in a cell culture bag are easily controlled. Provided is a system including a sac-like cell culture bag having a supply port for supplying a solution, such as a medium, to the inside and having a discharge port for discharging the solution, such as a medium, from the inside, wherein gate opening and closing means for opening and closing inner holes of the supply port and the discharge port are attached to the supply port and the discharge port, and the gate opening and closing means are controlled to easily exchange the medium or the like in the cell culture bag.

## Description

### {Technical Field}

The present invention relates to a medium exchange system for a cell culture bag.

### {Background Art}

In recent years, preparation of a large amount of cells for clinical use is demanded along with the progress in stem cell research and regenerative medicine, and a sack-like culture bag made of a gas-permeable material is often used to culture a large amount of cells, instead of culturing the cells in a flask or a petri dish (PTL 1).

### {Citation List}

### {Patent Literature}

{PTL 1} Japanese Unexamined Patent Application, Publication No. 2000-125848

### {Summary of Invention}

### {Technical Problem}

Work in an environment compliant with strict standards is demanded in the preparation of cells for clinical use. Therefore, it takes a great amount of labor and cost, such as changing into disposable working clothes, when the worker enters the work space. Thus, it is demanded to minimize the number of times the worker enters the work space and to perform remote operation of the work if possible.

The worker performs the work in a closed space, such as an isolator, through a glove-like isolation tool, and unlike in general work, the worker cannot directly touch the sample or the like. Therefore, an apparatus and a system with excellent operability are demanded.

The present invention has been made in view of the circumstances, and an object of the present invention is to provide a system that can easily exchange a culture medium or the like for cells in culture during culturing in a cell culture space.

### {Solution to Problem}

To attain the object, the present invention provides the following solutions.

An aspect of the present invention provides a medium exchange system including:
a sack-like cell culture bag including a supply port for supplying a solution, such as a medium, to the inside and a discharge port for discharging the solution, such as a medium, from the inside;
a vessel connected to the supply port, the vessel holding the solution, such as a medium, to be supplied to the supply port;
a negative pressure supply means connected to the discharge port, the negative pressure supply means supplying a negative pressure for discharging the solution, such as a medium, from the discharge port;
gate opening and closing means installed on the supply port and the discharge port, the gate opening and closing means opening and closing inner holes of the supply port and the discharge port; and
a control unit that controls the gate opening and closing means by remote operation.

According to the present aspect, the solution, such as a medium, can be remotely supplied to and discharged from the cell culture bag.

Another aspect of the present invention provides a medium exchange system including:
a sack-like cell culture bag including a supply port for supplying a solution, such as a medium, to the inside and a discharge port for discharging the solution, such as a medium, from the inside;
a sack-like trap bag including a supply port for supplying the solution, such as a medium, to the inside and a discharge port that is connected to the supply port of the cell culture bag and that is for discharging the solution, such as a medium, from the inside to the cell culture bag;
a vessel connected to the supply port of the trap bag, the vessel holding the solution, such as a solution, to be supplied to the trap bag;
a negative pressure supply means connected to the discharge port of the cell culture bag, the negative pressure supply means supplying a negative pressure for discharging the solution, such as a solution, from the cell culture bag;
gate opening and closing means installed on the supply port and the discharge port, the gate opening and closing means opening and closing inner holes of the supply port and the discharge port; and
a control unit that controls the gate opening and closing means by remote operation.

According to the present aspect, the solution, such as a medium, can be remotely supplied to and discharged from the cell culture bag. The risk of contamination of the medium or the like held in the vessel can be reduced.

The above-described aspects may further include a solution temperature control means for controlling the temperature of the solution, such as a medium, in the vessel.

This can prevent degradation of the solution, such as a medium, and can prevent the cells from being affected by a difference in temperature of the solution.

The above-described aspects may further include a bag holder that holds the cell culture bag and the trap bag or that holds the cell culture bag, the trap bag, and the gate opening and closing means, wherein
the control unit controls the bag holder by remote operation to control the gate opening and closing means.

As a result, the control unit can comprehensively control the gate opening and closing means.

The above-described aspects may further include a charge and discharge means for applying a charge to the cell culture bag and releasing the charge from the cell culture bag.

As a result, the cells can be attached to the inner surface of the cell culture bag, and the medium can also be exchanged in floating cell culture.

In the above-described aspects, the cell culture bag may include baffle parts that form a channel through which the solution flows inside.

This can easily spread the solution, such as a medium, everywhere in the cell bag.

Another aspect of the present invention provides a medium exchange system including:
a sack-like cell culture bag including a supply port for supplying a solution, such as a medium, to the inside and a discharge port for discharging the solution, such as a medium, from the inside;
a vessel connected to the supply port, the vessel holding the solution, such as a medium, to be supplied to the supply port;
a negative pressure supply means connected to the discharge port, the negative pressure supply means supplying a negative pressure for discharging the solution, such as a medium, from the discharge port;
gate opening and closing means installed on the supply port and the discharge port, the gate opening and closing means opening and closing inner holes of the supply port and the discharge port; and
an opening and closing switch that controls opening and closing of the inner holes by the gate opening and closing means.

### {Advantageous Effects of Invention}

According to the present invention, the medium of the cell culture bag can be exchanged by remote operation, and the number of times the worker enters the work space can be reduced. This can reduce the labor and cost, such as changing into disposable working clothes, and can lower the risk of contamination of the cell culture system by germs or the like.

The operation of work in a closed space, such as an isolator, can be simplified, and the risk of an operational error can be lowered.

### {Brief Description of Drawings}

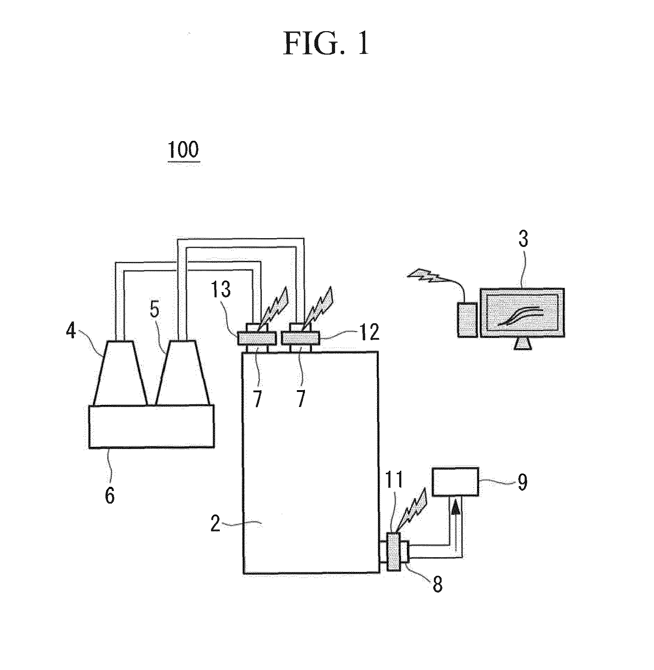

{Fig. 1}
   Fig. 1 is an explanatory view showing a schematic configuration of a medium exchange system according to a first embodiment of the present invention.
{Fig. 2A}
   Fig. 2A is an explanatory view showing a schematic configuration of a gate opening and closing means of the present invention.
{Fig. 2B}
   Fig. 2B is an explanatory view showing another schematic configuration of the gate opening and closing means of the present invention.
{Fig. 3}
   Fig. 3 is an explanatory view showing a schematic configuration of a modification of the medium exchange system according to the first embodiment of the present invention.
{Fig. 4}
   Fig. 4 is an explanatory view showing a schematic configuration of a medium exchange system according to a second embodiment of the present invention.
{Fig. 5}
   Fig. 5 is an explanatory view showing a schematic configuration of a medium exchange system according to a third embodiment of the present invention.
{Fig. 6A}
   Fig. 6A is an explanatory view showing a schematic configuration of a medium exchange system according to a fourth embodiment of the present invention and is a diagram showing an example of a bag holder according to the fourth embodiment.
{Fig. 6B}
   Fig. 6B is an explanatory view showing an example of the bag holder in the second embodiment of the present invention.
{Fig. 6C}
   Fig. 6C is an explanatory view showing an example of the bag holder in the third embodiment of the present invention.
{Fig. 7A}
   Fig. 7A is an explanatory view showing a schematic configuration of a medium exchange system according to a fifth embodiment of the present invention.
{Fig. 7B}
   Fig. 7B is an explanatory view showing an example of applying a charge and discharge means to the medium exchange system according to the second embodiment of the present invention.
{Fig. 7C}
   Fig. 7C is an explanatory view showing an example of applying the charge and discharge means to the medium exchange system according to the third embodiment of the present invention.
{Fig. 7D}
   Fig. 7D is an explanatory view showing a schematic configuration of a modification of the medium exchange system according to the fifth embodiment of the present invention.
{Fig. 8}
   Fig. 8 is an explanatory view showing a schematic configuration of a medium exchange system according to a sixth embodiment of the present invention.
{Fig. 9A}
   Fig. 9A is an explanatory view showing a schematic configuration of a medium exchange system according to a seventh embodiment of the present invention.
{Fig. 9B}
   Fig. 9B is an explanatory view showing an example of applying an opening and closing switch to the medium exchange system according to an embodiment of the present invention.
{Fig. 9C}
   Fig. 9C is an explanatory view showing an example of applying the opening and closing switch to a medium exchange system according to another embodiment of the present invention.
{Fig. 10A}
   Fig. 10A is an explanatory view showing a schematic configuration of the opening and closing switch of the present invention.
{Fig. 10B}
   Fig. 10B is an explanatory view showing another schematic configuration of the opening and closing switch of the present invention.
{Fig. 11A}
   Fig. 11A is an explanatory view showing a schematic configuration of a modification of the first embodiment of the present invention.
{Fig. 11B}
   Fig. 11B is an explanatory view showing a schematic configuration of a modification of the fourth embodiment of the present invention.
{Fig. 12A}
   Fig. 12A is an explanatory view showing a schematic configuration of a first example of a backflow prevention mechanism with a supply port, a discharge port, and a tube of the present invention.
{Fig. 12B}
   Fig. 12B is an explanatory view showing a schematic configuration of a second example of the backflow prevention mechanism with the supply port, the discharge port, and the tube of the present invention.
{Fig. 12C}
   Fig. 12C is an explanatory view showing a schematic configuration of a modification of the first example of the backflow prevention mechanism with the supply port, the discharge port, and the tube of the present invention.
{Fig. 12D}
   Fig. 12D is an explanatory view showing a schematic configuration of a modification of the second example of the backflow prevention mechanism with the supply port, the discharge port, and the tube of the present invention.

### {Description of Embodiments}

A cell culture apparatus 1 according to embodiments of the present invention will be described with reference to the drawings.

### (First Embodiment)

A medium exchange system 100 according to the present embodiment is a system used to culture adherent cells and has a configuration shown in Fig. 1.

A cell culture bag 2 is a sack-like bag made of a material with a high gas exchange performance, and processing (such as coating) for the cells to adhere is applied to the inner surface. The cell culture bag 2 includes supply ports 7 for supplying a solution, such as a medium, and a discharge port 8 for discharging the solution, such as a medium. Fig. 1 illustrates a view of the cell culture bag 2 from an upper surface direction.

The supply ports 7 are linked to a medium vessel 4 and a cleaning liquid vessel 5 through tubes or the like and can introduce a medium and a cleaning liquid (such as PBS (-)) into the culture bag 2 through the tubes or the like. Fig. 1 illustrates an example of a configuration in which the culture bag 2 includes two supply ports 7. One of the supply ports 7 is linked to the medium vessel 4, and the other is linked to the cleaning liquid vessel 5.

The discharge port 8 is linked to a negative pressure supply means 9 exemplified by a pump, through a tube or the like. The pump or the like can put the inside of the tube into a negative pressure state, and the discharge port 8 can discharge the solution, such as a medium, from the cell culture bag 2. The pump or the like can exchange information with a control unit 3 by remote operation, and ON/OFF of the switch of the pump is remotely controlled by an instruction from the control unit 3.

The discharge port 8 includes a gate opening and closing means 11, and the two supply ports 7 include gate opening and closing means 12 and 13. The gate opening and closing means 11, 12, and 13 can exchange information with the control unit 3 by remote operation, and the supply ports 7 and the discharge port 8 can be remotely opened and closed by an instruction from the control unit 3.

The medium vessel 4 and the cleaning liquid vessel 5 are provided on a solution temperature control means 6. The solution temperature control means 6 can exchange information with the control unit 3 by remote operation, and the temperature of the medium and the cleaning liquid can be remotely controlled by an instruction from the control unit 3. More specifically, the solution temperature control means 6 controls the medium at a low temperature (about 4°C) to prevent degradation of the medium or the like when the medium is not exchanged, and controls the medium at a temperature suitable for the cells (about 37°C) before the exchange of the medium.

Here, the cell culture bag 2 and the gate opening and closing means 11, 12, and 13 are arranged in an incubator that can control the environment in culture, and the other components, such as the medium vessel 4, the cleaning liquid vessel 5, and the pump, are arranged outside of the incubator.

Next, an example of a procedure of using the medium exchange system 100 according to the present embodiment to exchange the medium will be described.

The user of the present system first prepares the cell culture bag 2 filled with cells to be cultured and a medium and arranges the cell culture bag 2 in the incubator. The user connects the supply ports 7 to the medium vessel 4 and the cleaning liquid vessel 5 and connects the discharge port 8 to the pump through tubes. At the same time, the user sets the gate opening and closing means 11, 12, and 13. Here, the medium vessel 4, the cleaning liquid vessel 5, and the pump are installed outside of the incubator. The gate opening and closing means 11, 12, and 13 are in a closed state.

When the medium needs to be exchanged, the user first controls the solution temperature control means 6 through the control unit 3 to control the medium and the cleaning liquid at 37°C. The user further turns on the switch of the pump through the control unit 3 to set a negative pressure in the tube connected to the discharge port 8.

The control unit 3 puts the gate opening and closing means 11 into an open state. The negative pressure provides suction force to the medium in the cell culture bag 2 toward the pump, and the medium is discharged to the outside of the cell culture bag 2 from the discharge port 8.

The control unit 3 puts the gate opening and closing means 12 into the open state, and the negative pressure supplies the cleaning liquid in the cleaning liquid vessel 5 to the cell culture bag 2 from the supply port 7.

The control unit 3 puts the gate opening and closing means 12 into the closed state to cut off the supply of the cleaning liquid in the cleaning liquid vessel 5. Meanwhile, the gate opening and closing means 11 is in the open state, and the negative pressure discharges the cleaning liquid in the cell culture bag 2 to the outside of the cell culture bag 2 from the discharge port 8.

The control unit 3 puts the gate opening and closing means 13 into the open state, and the negative pressure supplies the medium in the medium vessel 4 to the cell culture bag 2 from the supply port 7. In the state that the cell culture bag 2 is filled with the medium, the control unit 3 puts the gate opening and closing means 11 and 13 into the closed state.

The control unit 3 turns off the switch of the pump to release the negative pressure in the tube connected to the discharge port 8. The control unit 3 further controls the solution temperature control means 6 to control the medium and the cleaning liquid at 4°C.

The user may use a remote monitoring system not shown to check the situation to operate the control timing of the gate opening and closing means 11, 12, and 13, or the control unit 3 may use a preset program to automatically control the control timing. In the automatic control, the control timing can be calculated based on the volume in the cell culture bag 2 and the suction speed of the pump.

Although the cleaning by the cleaning liquid is performed once in the above description, an arbitrary number of times of cleaning can be performed. The cleaning liquid vessel 5 may not be installed if cleaning is not necessary.

The waste liquid of the medium or the like sucked toward the pump is recovered in a waste liquid vessel (not shown) installed on the upstream of the pump.

Next, action of the gate opening and closing means will be described.

The supply ports 7 and the discharge port 8 are made of an elastic cylindrical material. The gate opening and closing means can apply force from the outside to collapse the cylindrical form, and an inner hole can be closely attached and shut. When the force from the outside is removed, the supply ports 7 and the discharge port 8 can return to the original cylindrical form by the elastic force, and the inner hole can be opened.

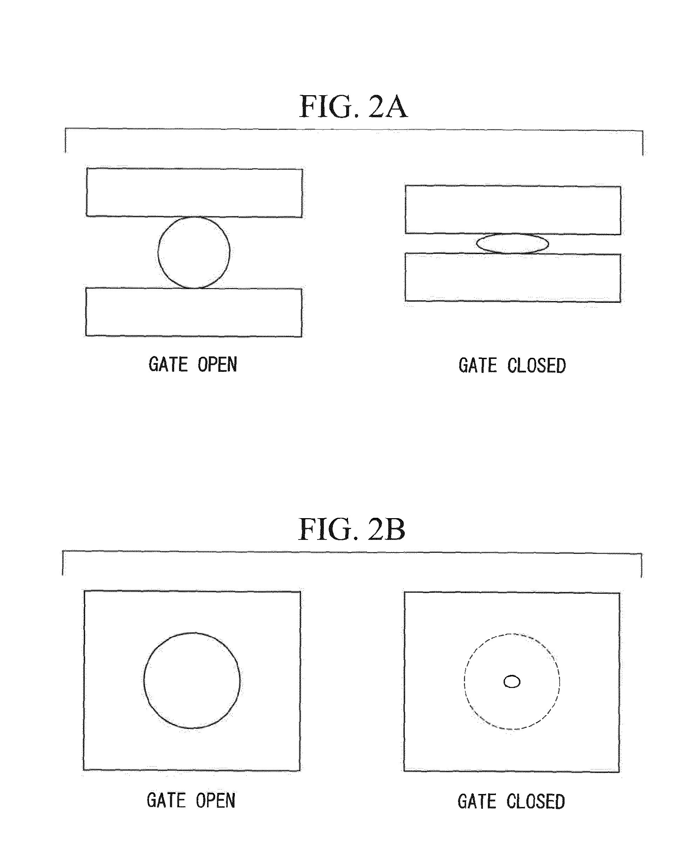

To apply the force from the outside by the gate opening and closing means, the supply ports 7 and the discharge port 8 can be placed between two plate-like members, and the plate-like members can be brought close to each other to collapse and press the supply ports 7 and the discharge port 8, as shown for example in Fig. 2A. An example of the gate opening and closing means includes a means in which the two plate-like members include electromagnets, and electrical signals can collapse and press or open the supply ports 7 and the discharge port 8.

The gate opening and closing means can be any means that can collapse and press the supply ports 7 and the discharge port 8 by applying force from the outside. A means including a hole for the supply ports 7 and the discharge port 8 as shown for example in Fig. 2B can be used in place of the two plate-like members, and the size of the hole can be controlled to collapse and press or open the supply ports 7 and the discharge port 8.

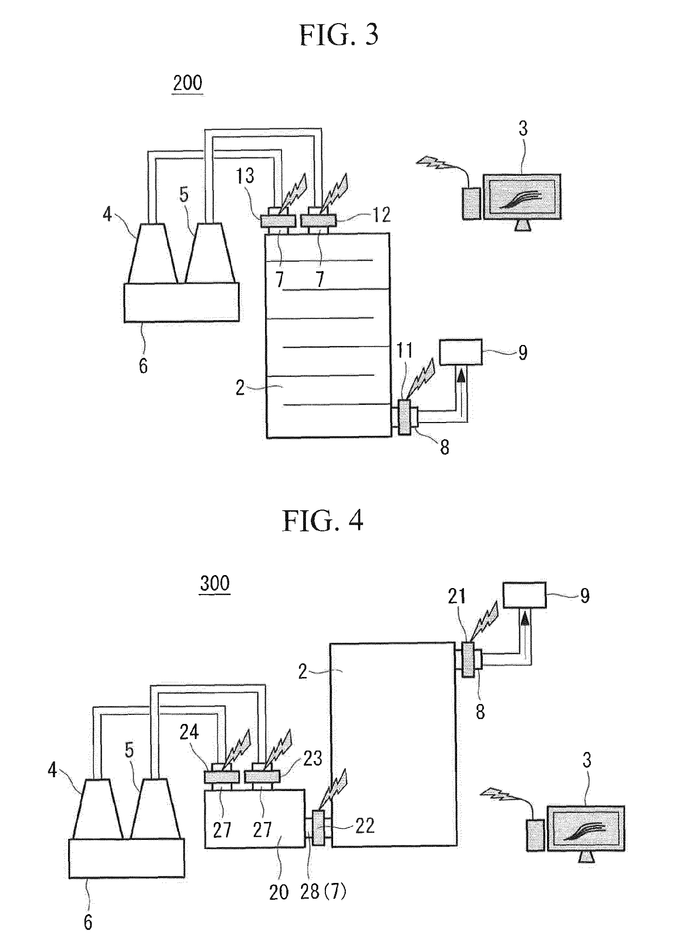

The cell culture bag 2 can be a cell culture bag 2 including baffle parts that form a channel inside as shown in Fig. 3. Using the cell culture bag can evenly deliver the solution everywhere in the bag.

### (Second Embodiment)

A medium exchange system 300 according to the present embodiment shown in Fig. 4 is a system used to culture adherent cells, and the medium exchange system according to the first embodiment further includes a trap bag 20. The trap bag 20 is a sack-like bag including supply ports 27 for supplying a solution, such as a medium, and a discharge port 28 for discharging the solution, such as a medium.

The supply ports 27 are linked to the medium vessel 4 and the cleaning liquid vessel 5 through tubes or the like. The medium and the cleaning liquid are introduced into the trap bag 20 from the supply ports 27 through tubes or the like. Fig. 4 illustrates a configuration in which the trap bag 20 includes two supply ports 27. One of the supply ports 27 is linked to the medium vessel 4, and the other is linked to the cleaning liquid vessel 5.

The discharge port 28 is linked and integrated with the supply port 7 of the cell culture bag 2 and is linked to the cell culture bag 2. The discharge port 8 of the cell culture bag 2 is linked to the negative pressure supply means 9 exemplified by a pump, through a tube or the like.

The two supply ports 27 of the trap bag 20, the discharge port 28 of the trap bag 20 (the supply port 7 of the cell culture bag 2), and the discharge port 8 of the cell culture bag 2 include gate opening and closing means 23, 24, 22, and 21, respectively. The gate opening and closing means 21, 22, 23, and 24 can exchange information with the control unit 3 by remote operation, and instructions from the control unit 3 can remotely open and close the supply ports 27 of the trap bag 20, the discharge port 28 of the trap bag 20 (the supply port 7 of the cell culture bag 2), and the discharge port 8 of the cell culture bag 2.

The other components have the same functions as the components of the first embodiment.

Here, the trap bag 20, the cell culture bag 2, and the gate opening and closing means 21, 22, 23, and 24 are arranged in the incubator that can control the environment in culture, and the other components are arranged outside of the incubator.

Next, a procedure of using the medium exchange system 300 according to the present embodiment to exchange the medium will be described.

The user of the present system first prepares the cell culture bag 2 filled with cells to be cultured and a medium and arranges the cell culture bag 2 in the incubator. The user links the supply port 7 of the cell culture bag 2 and the discharge port 28 of the trap bag 20. The two supply ports 27 of the trap bag 20 are connected to the medium vessel 4 and the cleaning liquid vessel 5, and the discharge port 8 of the cell culture bag 2 is connected to the pump through the tubes. Here, the medium vessel 4, the cleaning liquid vessel 5, and the pump are installed outside of the incubator. The gate opening and closing means 21, 22, 23, and 24 are in the closed state.

When the medium needs to be exchanged, the user first controls the solution temperature control means 6 through the control unit 3 to control the medium and the cleaning liquid at 37°C. The user further turns on the switch of the pump through the control unit 3 to set a negative pressure in the tube connected to the discharge port 8 of the cell culture bag 2.

The control unit 3 puts the gate opening and closing means 21 into the open state. The negative pressure provides suction force to the medium in the cell culture bag 2 toward the pump, and the medium is discharged to the outside of the cell culture bag 2 from the discharge port 8 of the cell culture bag 2.

The control unit 3 puts the gate opening and closing means 22 and 23 into the open state, and the negative pressure supplies the cleaning liquid in the cleaning liquid vessel 5 to the trap bag 20 from the supply port 27 of the trap bag 20. The cleaning liquid further passes through the discharge port 28 of the trap bag 20 (the supply port 7 of the cell culture bag 2) and is supplied to the cell culture bag 2.

The control unit 3 puts the gate opening and closing means 23 into the closed state to cut off the supply of the cleaning liquid in the cleaning liquid vessel 5. Meanwhile, the gate opening and closing means 21 is in the open state, and the negative pressure discharges the cleaning liquid in the cell culture bag 2 and the trap bag 20 to the outside of the cell culture bag 2 from the discharge port 8 of the cell culture bag 2.

The control unit 3 puts the gate opening and closing means 24 into the open state, and the negative pressure supplies the medium in the medium vessel 4 to the trap bag 20 from the supply port 27. The medium further passes through the discharge port 28 of the trap bag 20 (the supply port 7 of the cell culture bag 2) and is supplied to the cell culture bag 2.

In the state that the cell culture bag 2 is filled with the medium, the control unit 3 puts the gate opening and closing means 21, 22, and 24 into the closed state.

The control unit 3 turns off the switch of the pump to release the negative pressure in the tube connected to the discharge port 8 of the cell culture bag 2. The control unit 3 controls the solution temperature control means 6 to control the medium and the cleaning liquid at 4°C.

In the present embodiment, the installation of the trap bag 20 can trap the solution flown back from the cell culture bag 2, and the risk of the contamination of the solution in the medium vessel 4 and the cleaning liquid vessel 5 can be lowered.

As in the first embodiment, the cell culture bag 2 including the baffle parts that form a channel inside as shown in Fig. 3 may also be used.

### (Third Embodiment)

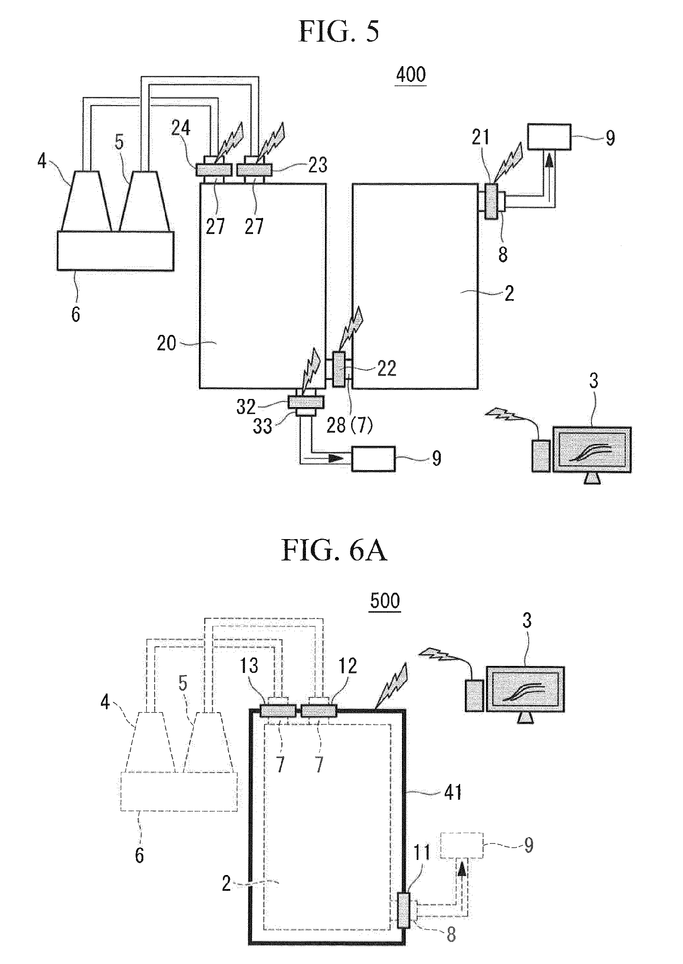

A medium exchange system 400 according to the present embodiment is a system used to culture adherent cells. As shown in Fig. 5, the trap bag 20 according to the second embodiment further includes a discharge port 33 and is linked to the negative pressure supply means 9 exemplified by a pump, through a tube or the like.

The supply ports 27 are linked to the medium vessel 4 and the cleaning liquid vessel 5 through tubes or the like. The medium and the cleaning liquid are introduced into the trap bag 20 through tubes or the like. Fig. 5 illustrates an example of a configuration in which the trap bag 20 includes two supply ports 27. One of the supply ports 27 is linked to the medium vessel 4, and the other is linked to the cleaning liquid vessel 5.

The discharge port 28 is linked and integrated with the supply port 7 of the cell culture bag 2 to link the trap bag 20 and the cell culture bag 2. The discharge port 8 of the cell culture bag 2 is linked to the negative pressure supply means 9 exemplified by a pump, through a tube or the like. The discharge port 33 of the trap bag 20 is linked to the negative pressure supply means 9 exemplified by the pump, through a tube or the like.

The discharge port 8 of the cell culture bag 2, the discharge port 28 of the trap bag 20 (the supply port 7 of the cell culture bag 2), the discharge port 33 of the trap bag 20, and the two supply ports 27 of the trap bag 20 include gate opening and closing means 21, 22, 32, 23, and 24, respectively. The gate opening and closing means 21, 22, 32, 23, and 24 can exchange information with the control unit 3 by remote operation, and instructions from the control unit 3 can remotely open and close the discharge port 8, the discharge port 28 (the supply port 7), the discharge port 33, and the supply ports 27.

The other components have the same functions as the components of the second embodiment.

Here, the trap bag 20, the cell culture bag 2, and the gate opening and closing means 21, 22, 32, 23, and 24 are arranged in the incubator that can control the environment in culture, and the other components are arranged outside of the incubator.

Next, an example of a procedure of using the medium exchange system 400 according to the present embodiment to exchange the medium will be described.

The user of the present system first prepares the cell culture bag 2 filled with cells to be cultured and a medium and arranges the cell culture bag 2 in the incubator. The user links the supply port 7 of the cell culture bag 2 and the discharge port 28 of the trap bag 20. The supply ports 27 of the trap bag 20 are connected to the medium vessel 4 and the cleaning liquid vessel 5, and the discharge port 8 of the cell culture bag 2 and the discharge port 33 of the trap bag 20 are connected to the pump through the tubes. Here, the medium vessel 4, the cleaning liquid vessel 5, and the pump are installed outside of the incubator. The gate opening and closing means 21, 22, 32, 23, and 24 are in the closed state.

When the medium needs to be exchanged, the user first uses the solution temperature control means 6 through the control unit 3 to control the medium and the cleaning liquid at 37°C. The user further turns on the switch of the pump through the control unit 3 to set a negative pressure in the tubes connected to the discharge ports 8 and 33.

The control unit 3 puts the gate opening and closing means 21 into the open state. The negative pressure provides suction force to the medium in the cell culture bag 2 toward the pump, and the medium is discharged to the outside of the cell culture bag 2 from the discharge port 8 of the cell culture bag 2.

Next (or at the same time), the control unit 3 puts the gate opening and closing means 32 and 23 into the open state, and the negative pressure supplies the cleaning liquid in the cleaning liquid vessel 5 to the trap bag 20 from the supply port 27. The gate opening and closing means 32 and 23 are put into the closed state when the trap bag 20 is filled with the cleaning liquid.

The control unit 3 puts the gate opening and closing means 22 into the open state to move the cleaning liquid in the trap bag 20 into the cell culture bag 2 and further discharges the cleaning liquid from the discharge port 8 of the cell culture bag 2 to clean the inside of the cell culture bag 2.

The control unit 3 puts the gate opening and closing means 22 into the closed state and puts the gate opening and closing means 32 and 24 into the open state, and the negative pressure supplies the medium in the medium vessel 4 into the trap bag 20 from the supply port 27 of the trap bag 20. The gate opening and closing means 32 and 24 are put into the closed state when the trap bag 20 is filled with the medium.

The control unit 3 puts the gate opening and closing means 22 into the open state to move the medium in the trap bag 20 into the cell culture bag 2 and puts the gate opening and closing means 22 and 21 into the closed state when the cell culture bag 2 is filled with the medium.

The control unit 3 turns off the switch of the pump to release the negative pressure in the tube connected to the discharge ports 8 and 33. The control unit 3 controls the solution temperature control means 6 to control the medium and the cleaning liquid at 4°C.

In the present embodiment, the trap bag 20 can hold the solution before the solution is supplied to the cell culture bag 2, and this can lower the risk of contamination of the solution in the medium vessel 4 and the cleaning liquid vessel 5 due to the solution flown back from the cell culture bag 2.

As in the first embodiment, the cell culture bag 2 including the baffle parts that form a channel inside as shown in Fig. 3 may also be used.

### (Fourth Embodiment)

A medium exchange system 500 according to the present embodiment includes a bag holder 41 that holds the cell culture bag 2 according to the first embodiment as shown in Fig. 6A.

The bag holder 41 includes the gate opening and closing means 11, 12, and 13, and the cell culture bag 2 can be set in the bag holder 41 to attach the gate opening and closing means 11, 12, and 13 to the supply ports 7 and the discharge port 8 of the cell culture bag 2. The bag holder 41 can exchange information with the control unit 3 by remote operation, and instructions from the control unit 3 can remotely control the gate opening and closing means 11, 12, and 13 to open and close the supply ports 7 and the discharge port 8 of the cell culture bag 2. Although the control unit 3 separately controls the gate opening and closing means 11, 12, and 13 in the first embodiment, the control unit 3 can control the bag holder 41 to comprehensively control the gate opening and closing means 11, 12, and 13 in the present embodiment. For example, the bag holder 41 includes a control means in bag holder (not shown), and the control means in bag holder can exchange information with the control unit 3 by remote operation. The control means in bag holder is configured to identify the gate opening and closing means included in the bag holder and to control the opening and closing of the gate opening and closing means based on information from the control unit 3.

The functions equivalent to the first embodiment are provided, except for the control of the gate opening and closing means 11, 12, and 13 through the bag holder 41.

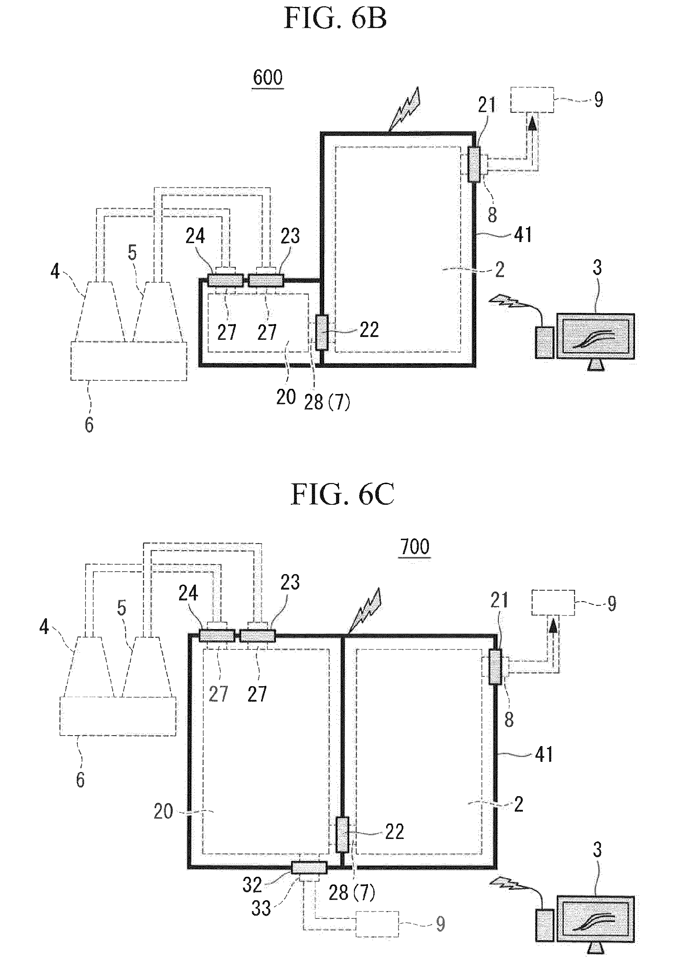

The bag holder 41 may be a bag holder 41 that holds the cell culture bag 2 and the trap bag 20 in the second and third embodiments as shown in Figs. 6B and 6C. In this case, the bag holder 41 includes the gate opening and closing means, and the control unit 3 can comprehensively control the gate opening and closing means through the bag holder 41.

As in the first embodiment, the cell culture bag 2 may be a cell culture bag 2 including the baffle parts that form a channel inside as shown in Fig. 3.

### (Fifth Embodiment)

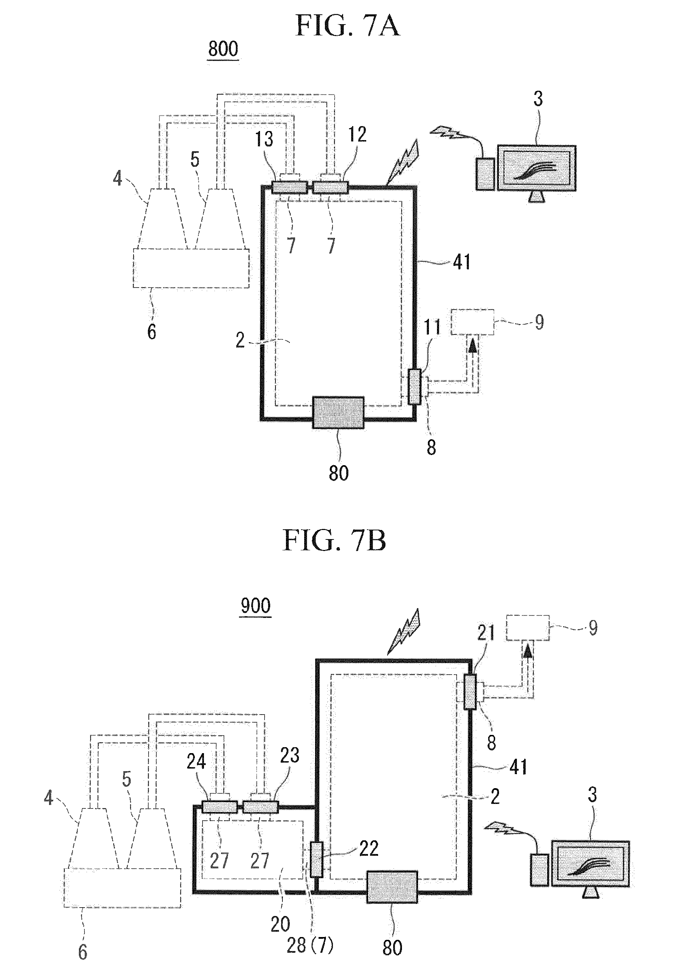

In a medium exchange system 800 according to the present embodiment, the medium exchange system according to the fourth embodiment further includes a charge and discharge means 80 that can apply a charge to the cell culture bag 2 and release the charge from the cell culture bag 2, as shown in Fig. 7A. As a result, the medium can also be exchanged in floating cell culture. More specifically, the cells have a negative charge, and a positive charge can be applied to the cell culture bag 2 to temporarily attach the floating cells to the inner surface of the cell culture bag 2. This can prevent discharge of the cells from the discharge port 8 when the solution, such as a medium, is exchanged. When the medium is not exchanged, the charge and discharge means 80 can release the charge applied to the cell culture bag 2.

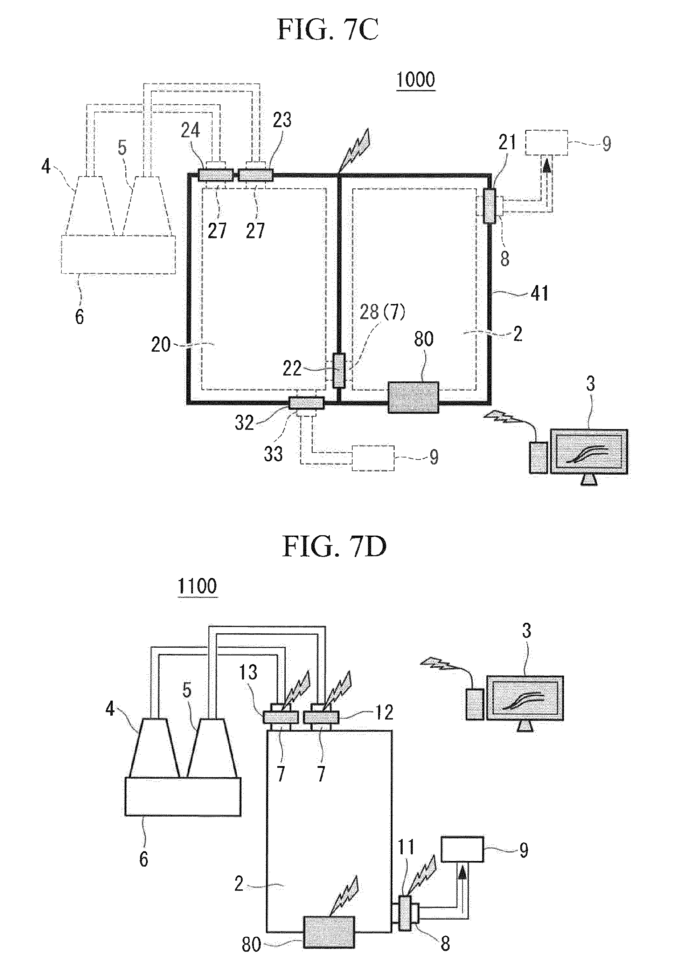

Fig. 7B illustrates an example in which the charge and discharge means is applied to the medium exchange system according to the second embodiment, and Fig. 7C illustrates an example in which the charge and discharge means is applied to the medium exchange system according to the third embodiment.

As in the first embodiment, the cell culture bag 2 may be a cell culture bag 2 including the baffle parts that form a channel inside as shown in Fig. 3.

The charge and discharge means 80 may be installed on the bag holder 41, or the control unit 3 may independently control the charge and discharge means 80 in a mode without the bag holder 41 as shown in Fig. 7D.

### (Sixth Embodiment)

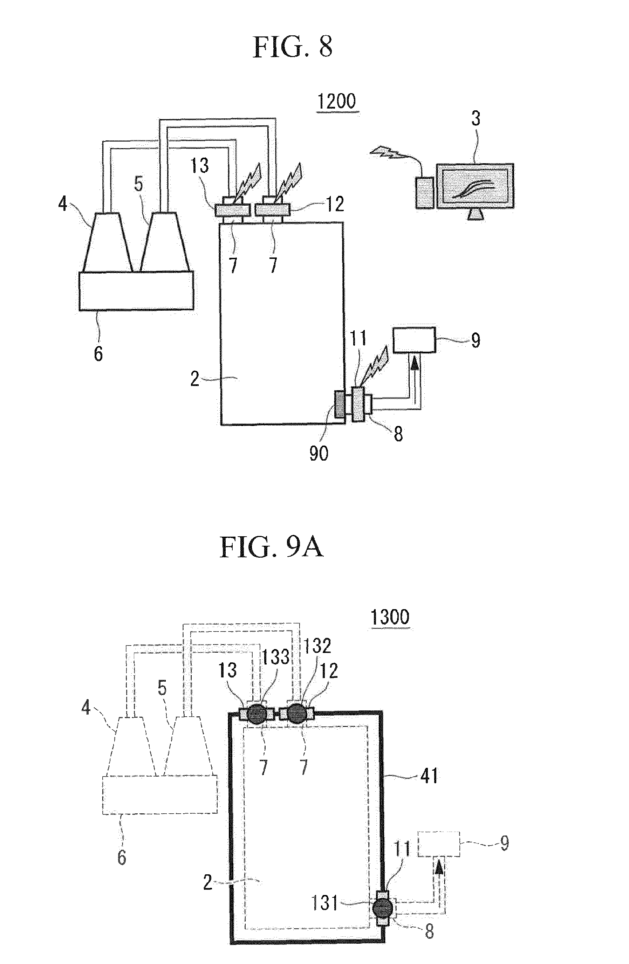

In a medium exchange system 1200 according to the present embodiment, the discharge port 8 of the cell culture bag 2 according to the embodiments includes a filter 90 that can trap the cells. As a result, the medium can also be exchanged in floating cell culture. Fig. 8 illustrates an example of a configuration in which the medium exchange system according to the first embodiment includes the filter 90. The discharge port 8 of the cell culture bag 2 is provided with the filter 90 that allows the solution, such as a medium, to pass through, but that does not allow the cells to pass through. It is more preferable that the filter 90 traps only normal cells, allowing abnormal cells to pass through.

Although Fig. 8 illustrates an example corresponding to the medium exchange system according to the first embodiment, the example can also be similarly applied to the medium exchange systems of the other embodiments. In the present embodiment, the cell culture bag 2 including the baffle parts that form a channel inside as shown in Fig. 3 may also be used.

### (Seventh Embodiment)

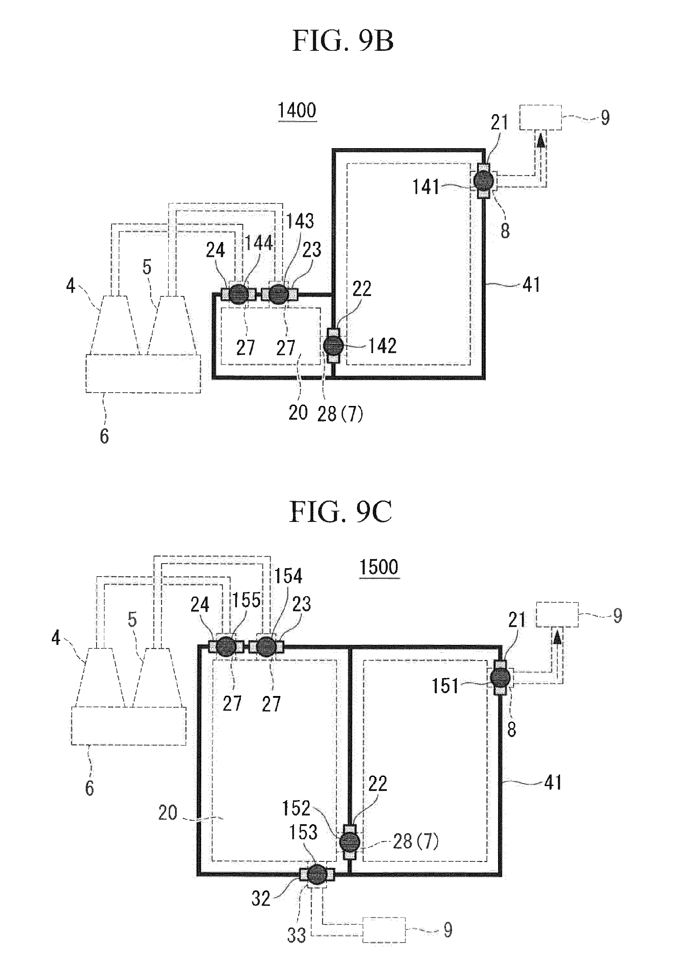

In a medium exchange system of the present embodiment, the gate opening and closing means according to the embodiments include opening and closing switches, and the worker does not remotely operate the gate opening and closing means through the control unit. The system is usable when the worker uses an isolator or the like to perform manual operation. Fig. 9 illustrates an example corresponding to the fourth embodiment.

A medium exchange system 1300 according to the present embodiment exemplified in Fig. 9A will be described.

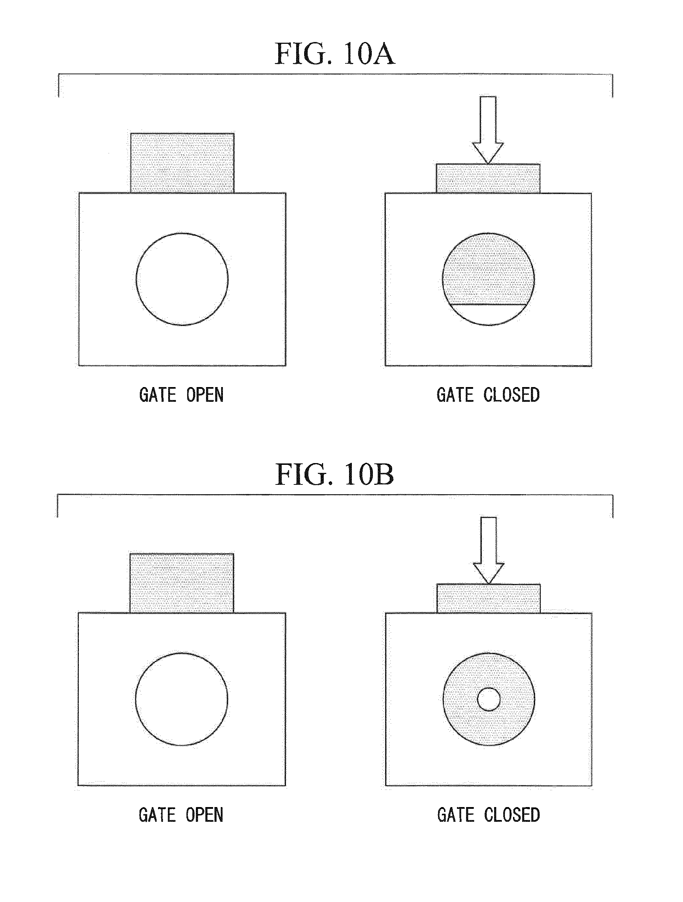

Switches of opening and closing switches 131, 132, and 133 can be manually changed to open and close the gate opening and closing means. As illustrated in Figs. 10A and 10B, the opening and closing switches are, for example, press buttons. The gate enters an open (or closed) state when the button is pressed once, and the state is maintained. The gate enters a closed (or open) state when the button is pressed one more time. In the example illustrated in Fig. 10A, the button is pressed to insert a shutter-like plate to cut off a through hole, and the supply port or the discharge port in the through hole is collapsed and pressed to shut the inner hole. The shutter-like plate exits from the through hole when the button is pressed one more time, and the supply port or the discharge port returns to the original state by the elastic force to open the inner hole. In the example illustrated in Fig. 10B, the button is pressed to reduce the diameter of the through hole, and the supply port or the discharge port in the through hole is collapsed and pressed to shut the inner hole. The diameter of the through hole is restored when the button is pressed one more time, and the supply port or the discharge port returns to the original state by the elastic force to open the inner hole.

Next, an example of a procedure of using the medium exchange system 1300 according to the present embodiment to exchange the medium will be described.

The user of the present system first prepares the cell culture bag 2 filled with the medium and the cells to be cultured for which the medium needs to be exchanged. The user connects the supply ports 7 of the cell culture bag 2 to the medium vessel 4 and the cleaning liquid vessel 5 and connects the discharge port 8 of the cell culture bag 2 to the pump through tubes or the like. At the same time, the user sets the gate opening and closing means 11, 12, and 13. Here, the gate opening and closing means 11, 12, and 13 are in the closed state.

The user first controls the medium and the cleaning liquid at 37°C through the solution temperature control means 6. The user further turns on the switch of the pump to set a negative pressure in the tube connected to the discharge port 8.

When the user manually turns on the opening and closing switch 131 to put the gate opening and closing means 11 into the open state, the negative pressure provides suction force to the medium in the cell culture bag 2 toward the pump, and the medium is discharged to the outside of the cell culture bag 2 from the discharge port 8.

When the user manually turns on the opening and closing switch 132 to put the gate opening and closing means 12 into the open state, the negative pressure supplies the cleaning liquid in the cleaning liquid vessel 5 to the cell culture bag 2 from the supply port 7.

When the user manually turns off the opening and closing switch 132 to put the gate opening and closing means 12 into the closed state, the supply of the cleaning liquid in the cleaning liquid vessel 5 is cut off. Meanwhile, the gate opening and closing means 11 is in the open state, and the negative pressure discharges the cleaning liquid in the cell culture bag 2 to the outside of the cell culture bag 2 from the discharge port 8.

When the user manually turns on the opening and closing means 133 to put the gate opening and closing means 13 into the open state, the negative pressure supplies the medium in the medium vessel 4 to the cell culture bag 2 from the supply port 7. In the state that the cell culture bag 2 is filled with the medium, the user manually turns off the opening and closing switches 131 and 133 to put the gate opening and closing means 11 and 13 into the closed state.

The user turns off the switch of the pump to release the negative pressure in the tube connected to the discharge port 8. The user further controls the medium and the cleaning liquid at 4°C through the solution temperature control means 6.

The example of the procedure using the medium exchange system 1300 shown in Fig. 9A has been described as above. The systems according to the other embodiments of the present invention can include the opening and closing switches provided on the gate opening and closing means in the medium exchange systems according to the embodiments as shown for example in Figs. 9B and 9C, and the user can manually exchange the medium instead of the remote operation.

The opening and closing of the gate opening and closing means is switched every time the opening and closing switch is pressed in the present embodiment. The gate opening and closing means may enter the open (or closed) state only when the switch is being pressed, and the gate opening and closing means may enter the closed (or open) state when the opening and closing switch is restored by the release of the pressing force.

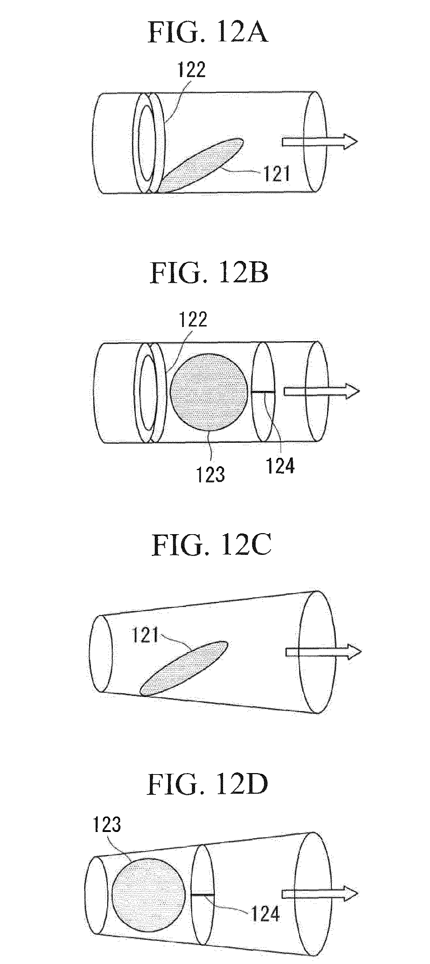

The supply ports and the discharge ports according to the embodiments may include a mechanism for preventing backflow of the solution. Each tube may also include a mechanism for preventing backflow of the solution. This can reduce the risk of contamination of the cell culture bag, the medium vessel, and the cleaning liquid vessel. The mechanism for preventing backflow in the present invention is a mechanism with a structure that permits flow of the solution, such as a medium, only in the direction of the flow to the negative pressure supply means 9. For example, as shown in Fig. 12A, the mechanism can include: a valve 121 with a diameter that can block the inner diameter of the supply port, the discharge port, or the tube, the valve 121 capable of falling down only in one direction; and a convex member 122 in the opposite direction of the valve 121, the convex member 122 stopping the valve 121 and preventing the valve 121 from falling down, thereby blocking the inner diameter of the supply port, the discharge port, or the tube. The convex member 122 may not be ring-shaped as shown in Fig. 12A, and any member with a structure that prevents the valve 121 from falling down is possible.

A ball 123 may be used in place of the valve as shown in Fig. 12B. In this case, when there is a backflow, the convex member 122 stops the ball 123 to block the channel to cut off the backflow. When the flow is normal, a trap member 124 stops the progress of the ball 123, and the solution flows from the gap between the ball 123 and the inner diameter of the supply port, the discharge port, or the tube. The trap member 124 is not limited to the illustrated member, and any member with a structure that stops the progress of the ball 123 and that allows the solution to pass through is possible.

Figs. 12C and 12D illustrate modifications of Figs. 12A and 12B without the convex member 122, wherein the inner diameter of part of the supply port, the discharge port, and the tube is gradually extended. In this case, when there is a backflow, the valve 121 or the ball 123 is stuck inside of the supply port, the discharge port, or the tube, and the channel is blocked.

In the embodiments, the user may use a remote monitoring system not shown to check the situation to operate the control timing of the gate opening and closing means through the control unit 3, or the control unit 3 may use a preset program to automatically control the control timing. In the automatic control, the control timing can be calculated based on the type (volume) of the used cell culture bag 2 and the suction speed of the pump. According to the automatic control, a mistake is not made in the order and timing of switching the gate opening and closing means.

Although the cleaning by the cleaning liquid is performed once in the embodiments, an arbitrary number of times of cleaning can be performed. The cleaning liquid vessel 5 and the like may not be installed if cleaning is not necessary.

The waste liquid of the medium or the like sucked toward the pump is recovered in a waste liquid vessel installed on the upstream of the pump.

The gate opening and closing means illustrated in the above-described embodiments apply force from the outside to collapse and press the supply port 7 and the discharge port 8 of the cell culture bag 2. Valves may be provided in the supply port 7 and the discharge port 8 of the cell culture bag 2, and the gate opening and closing means may operate the valves to control the opening and closing of the supply port 7 and the discharge port 8 of the cell culture bag 2.

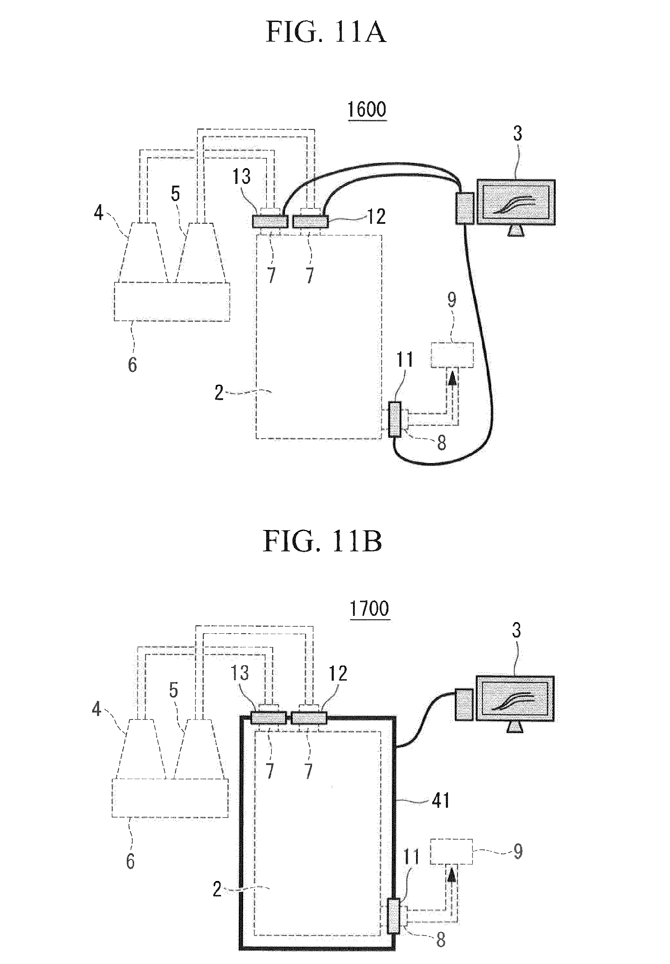

In a mode in which the control unit 3 wirelessly and remotely controls the exchange of the medium in the embodiments, the control unit 3 may remotely control the exchange of the medium through a wire, instead of in a wireless manner, as shown for example in Figs. 11A and 11B. The control unit 3 may control each gate opening and closing means through a wire as shown in Fig. 11A, or the control unit 3 may control each gate opening and closing means via the bag holder 41 through a wire as shown in Fig. 11B. The control unit 3 may similarly control each gate opening and closing means through a wire in the embodiments other than the embodiments illustrated in Figs. 11A and 11B.

### {Reference Signs List}

| | |
|---|---|
| 2 | cell culture bag |
| 3 | control unit |
| 4 | medium vessel |
| 5 | cleaning liquid vessel |
| 6 | solution temperature control means |
| 7, 27 | supply ports |
| 8, 28, 33 | discharge ports |
| 9 | negative pressure supply means |
| 11, 12, 13, 21, 22, 23, 24, 32 | gate opening and closing means |
| 20 | trap bag |
| 41 | bag holder |
| 80 | charge and discharge means |
| 90 | filter |
| 131, 132, 133, 141, 142, 143, 144, 151, 152, 153, 154, 155 | opening and closing switches |

## Claims

1. A medium exchange system comprising:
a sack-like cell culture bag including a supply port for supplying a solution to the inside and a discharge port for discharging the solution from the inside;
a vessel connected to the supply port, the vessel holding the solution to be supplied to the supply port;
a negative pressure supply means connected to the discharge port, the negative pressure supply means supplying a negative pressure for discharging the solution from the discharge port;
gate opening and closing means installed on the supply port and the discharge port, the gate opening and closing means opening and closing inner holes of the supply port and the discharge port; and
a control unit that controls the gate opening and closing means by remote operation.

2. A medium exchange system comprising:
a sack-like cell culture bag including a supply port for supplying a solution to the inside and a discharge port for discharging the solution from the inside;
a sack-like trap bag including a supply port for supplying the solution to the inside and a discharge port that is connected to the supply port of the cell culture bag and that is for discharging the solution from the inside to the cell culture bag;
a vessel connected to the supply port of the trap bag, the vessel holding the solution to be supplied to the trap bag;
a negative pressure supply means connected to the discharge port of the cell culture bag, the negative pressure supply means supplying a negative pressure for discharging the solution from the cell culture bag;
gate opening and closing means installed on the supply port and the discharge port of the cell culture bag as well as the supply port and the discharge port of the trap bag, the gate opening and closing means opening and closing inner holes of the supply ports and the discharge ports; and
a control unit that controls the gate opening and closing means by remote operation.

3. The medium exchange system according to claim 2,
wherein the trap bag further includes a discharge port connected to the negative pressure supply means.

4. The medium exchange system according to any of claims 1 to 3,
wherein the control unit controls the negative pressure supply means by remote operation.

5. The medium exchange system according to any of claims 1 to 4, further comprising
a solution temperature control means for controlling the temperature of the solution in the vessel, wherein
the control unit controls the solution temperature control means by remote operation.

6. The medium exchange system according to any of claims 1 to 5, further comprising
a bag holder that holds the cell culture or that holds the cell culture bag and the trap bag and that comprises the gate opening and closing means,
wherein the control unit controls the bag holder by remote operation to control the gate opening and closing means.

7. The medium exchange system according to any of claims 1 to 6, further comprising
a charge and discharge means for applying a charge to the cell culture bag and releasing the charge from the cell culture bag,
wherein the control unit controls the charge and discharge means by remote operation.

8. The medium exchange system according to claim 7, wherein the bag holder holds the charge and discharge means, and the control unit controls the bag holder by remote operation to control the charge and discharge means.

9. The medium exchange system according to any of claims 1 to 8,
wherein the cell culture bag includes baffle plates that form a channel through which the solution flows inside.

10. The medium exchange system according to any of claims 1 to 9,
wherein the discharge port of the cell culture bag comprises a filter that can trap cells.

11. A medium exchange system comprising:
a sack-like cell culture bag including a supply port for supplying a solution to the inside and a discharge port for discharging the solution from the inside;
a vessel connected to the supply port, the vessel holding the solution to be supplied to the supply port;
a negative pressure supply means connected to the discharge port, the negative pressure supply means supplying a negative pressure for discharging the solution from the discharge port;
gate opening and closing means installed on the supply port and the discharge port, the gate opening and closing means opening and closing inner holes of the supply port and the discharge port; and
an opening and closing switch that controls opening and closing of the inner holes by the gate opening and closing means.

12. A medium exchange system comprising:
a sack-like cell culture bag including a supply port for supplying a solution to the inside and a discharge port for discharging the solution from the inside;
a sack-like trap bag including a supply port for supplying the solution to the inside and a discharge port that is connected to the supply port of the cell culture bag and that is for discharging the solution from the inside to the cell culture bag;
a vessel connected to the supply port of the trap bag, the vessel holding the solution to be supplied to the trap bag;
a negative pressure supply means connected to the discharge port of the cell culture bag, the negative pressure supply means supplying a negative pressure for discharging the solution from the cell culture bag;
gate opening and closing means installed on the supply port and the discharge port of the cell culture bag as well as the supply port and the discharge port of the trap bag, the gate opening and closing means opening and closing inner holes of the supply ports and the discharge ports; and
an opening and closing switch that controls opening and closing of the inner holes by the gate opening and closing means.

13. The medium exchange system according to claim 12, wherein the trap bag further includes a discharge port connected to the negative pressure supply means.

14. The medium exchange system according to any of claims 11 to 13, further comprising
a bag holder that holds the cell culture bag or that holds the cell culture bag and the trap bag and that comprises the gate opening and closing means.
